# EUROPEAN PATENT SPECIFICATION

(11) **EP 3 718 524 B1**
(45) Date of publication and mention of the grant of the patent: **02.11.2022**
(21) Application number: 18884789.1
(22) Date of filing: 16.11.2018
(51) Int. Cl.: A61F 13/537, A61F 13/511, A61F 13/512, A61F 13/53, A61F 13/535

(54) **ABSORBENT ARTICLE**
ABSORBIERENDER ARTIKEL
ARTICLE ABSORBANT

(30) Priority: 29.11.2017 JP 2017229369
(43) Date of publication of application: 07.10.2020
(73) Proprietor: Kao Corporation, Chuo-Ku Tokyo 103-8210 (JP)
(72) Inventor: KUWAHATA, Kohei, Haga-gun Tochigi 321-3497 (JP)
(74) Representative: Vossius & Partner Patentanwälte Rechtsanwälte mbB
(86) International application number: PCT/JP2018/042537
(87) International publication number: WO 2019/107193

(56) References cited:
- WO-A1-2011/142272
- JP-A- 2010 017 342
- JP-A- 2012 061 014
- JP-A- 2012 120 584
- JP-A- 2012 120 584
- JP-A- 2013 255 565
- JP-A- 2014 018 647
- JP-A- 2017 148 141
- JP-A- 2017 148 141

## Description

### Technical Field

The present invention relates to an absorbent article such as a disposable diaper.

### Background Art

Conventionally, improving absorption performance for loose stools has been one technical problem of absorbent articles. Loose stools are often the case particularly with babies that are a few months old, and have high viscosities and are likely to flow. Accordingly, loose stools are unlikely to be absorbed into a diaper, and loose stools remaining on the topsheet may flow along the topsheet and leak to the outside.

There are known absorbent articles in which a liquid permeable intermediate sheet is provided between a topsheet and an absorbent member in order to improve absorbing performance of the absorbent member. The intermediate sheet in this case is a sheet that forms a layer different from the absorbent member, and is called a sublayer or a second sheet. For example, JP 2010-17342A discloses an absorbent article in which an uneven second sheet is provided between an absorbent member and a topsheet made of a liquid permeable material, the second sheet having a plurality of recessed grooves on both the front face side and the back face side thereof. Furthermore, JP 2012-61014A discloses an absorbent article in which an uneven second sheet is provided between an absorbent member having an absorbent member core and a liquid permeable topsheet, the second sheet having at least either a plurality of projections projecting toward the topsheet or a plurality of recesses recessed toward the absorbent member core.

Further related art is shown in JP 2017-148141 and WO 2011/142272 A1.

### Summary of Invention

The present invention is directed to an absorbent article as defined in independent claim 1.

### Brief Description of Drawings

[Fig. 1] Fig. 1 is a partially cutaway plan view as viewed from a skin-facing surface side of a disposable diaper that is an embodiment of an absorbent article according to the present invention, and is a plan view in an opened state in which the diaper is spread out in a planar shape with elastic members of respective portions in a stretched state.
[Fig. 2] Fig. 2 is a horizontal cross-sectional view schematically showing a cross-section taken along the line II-II of Fig. 1.
[Fig. 3] Fig. 3 is a perspective view schematically showing a main portion of an intermediate sheet included in the disposable diaper shown in Fig. 1.
[Fig. 4] Fig. 4 is a partially enlarged cross-sectional view enlarging part of a horizontal cross-section of the intermediate sheet shown in Fig. 3.
[Fig. 5] Fig. 5 is a perspective view schematically showing a main portion of a topsheet included in the disposable diaper shown in Fig. 1.
[Fig. 6] Fig. 6 is a partially enlarged plan view as viewed from a skin-facing surface side of the disposable diaper shown in Fig. 1.
[Fig. 7] Fig. 7 is a horizontal cross-sectional view of a disposable diaper that is another embodiment of an absorbent article according to the present invention (a view corresponding to Fig. 2).
[Fig. 8] Fig. 8 is a plan view as viewed from a non-skin-facing surface side of an intermediate sheet included in the disposable diaper shown in Fig. 7.

### Description of Embodiments

JP 2010-17342A and JP 2012-61014A make no mention whatsoever of body pressure or the like that will be applied to an absorbent article when a baby that is a few months old wears the absorbent article. The absorbent articles disclosed in JP 2010-17342A and JP 2012-61014A include an uneven second sheet, but JP 2010-17342A and JP 2012-61014A make no mention whatsoever of the properties of the uneven second sheet when the absorbent articles are worn.

Accordingly, the present invention relates to an absorbent article that can effectively absorb loose stools, and suppresses attachment of loose stools to the wearer's skin.

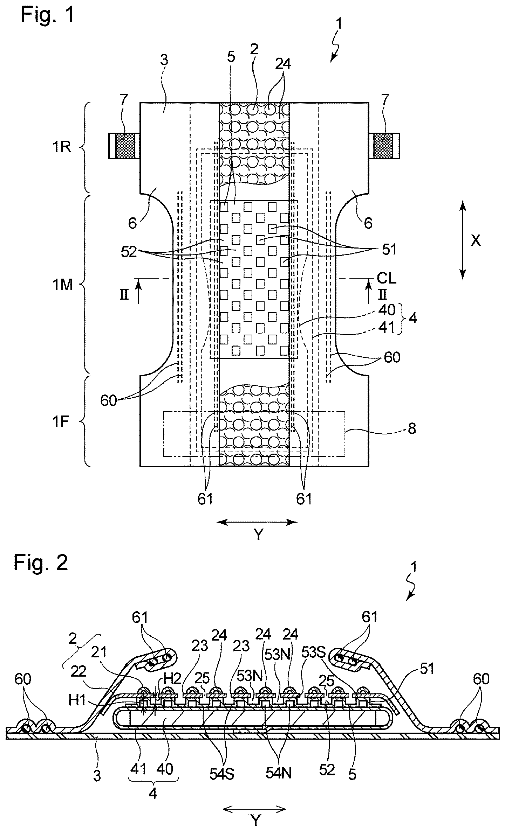

Hereinafter, the absorbent article according to the present invention will be described based on a preferred embodiment thereof with reference to the drawings. Figs. 1 and 2 show a disposable diaper 1 that is an embodiment of an absorbent article according to the present invention. The diaper 1 has a front portion 1F that is located on the wearer's ventral side and a rear portion 1R that is located on the dorsal side when worn, and a crotch portion 1M that is positioned between these portions, and has a longitudinal direction X extending from the front portion 1F via the crotch portion 1M to the rear portion 1R and corresponding to a front-rear direction of a wearer, and a lateral direction Y orthogonal to the longitudinal direction X. When the diaper 1 is bisected in the longitudinal direction X, the crotch portion 1M covers an area corresponding to 25 to 60%, and preferably 30 to 50%, of the entire length of the diaper 1 about a bisector CL extending in the lateral direction Y.

As shown in Figs. 1 and 2, the diaper 1 includes a liquid permeable topsheet 2 that may be in contact with the wearer's skin when worn, a liquid impermeable or water repellent backsheet 3, an absorbent member 4 that is arranged between the sheets 2 and 3, and an intermediate sheet 5 that is arranged between the topsheet 2 and the absorbent member 4. In a plan view as shown in Fig. 1, the diaper 1 has the shape of a longitudinally oblong hourglass that is inwardly narrowed substantially at the center in the longitudinal direction X of the crotch portion 1M and is oblong in one direction, that is, the longitudinal direction X.

Note that, in this specification, "skin-facing surface" is a surface that faces the wearer's skin, that is, the side relatively close to the wearer's skin when the absorbent article is worn, of the absorbent article or its constituent member (e.g., the topsheet 2), and "non-skin-facing surface" is a surface that faces the side (clothing side) opposite to the skin, that is, the side relatively distant from the wearer's skin when an absorbent article is worn, of the absorbent article or its constituent member. Note that "when worn" means a normal and proper worn position, that is, a state in which the correctly worn position of the absorbent article is maintained, and does not encompass a state in which the absorbent article is not in the correctly worn position.

Each of the topsheet 2 and the backsheet 3 has a size larger than that of the absorbent member 4, and extends outward from the outer edge of the absorbent member 4. The topsheet 2 will be described later in detail. The backsheet 3 forms an outer shape of the diaper 1 in its open and uncontracted state as shown in Fig. 1. As the backsheet 3, various types of backsheets that are conventionally used in this type of absorbent article can be used without particular limitation. Examples of the backsheet 3 include a resin film, a laminate of a resin film and a nonwoven fabric, and the like. The backsheet 3 may have, for example, a form in which a liquid impermeable film sheet is used alone or a form in which an outer sheet is stacked and arranged on the non-skin-facing surface, that is, an outer surface side of the film sheet, and the outer sheet may be a nonwoven fabric, for example.

In a plan view as shown in Fig. 1, the absorbent member 4 has a shape that is oblong in the longitudinal direction X, and extends from the front portion 1F to the rear portion 1R. The absorbent member 4 includes a liquid retentive absorbent core 40 containing an absorbent material, and a core-wrap sheet 41 that is wrapped around the skin-facing surface and the non-skin-facing surface of the absorbent core 40. The absorbent core 40 and the core-wrap sheet 41 are joined by a known joining means such as a hot melt adhesive.

The absorbent core 40 has a monolayer structure, and, in a plan view as shown in Fig. 1, the absorbent core 40 has an hourglass shape that is inwardly narrowed substantially at the center in the lengthwise direction, that is, the longitudinal direction X. The absorbent core 40 is formed by stacking fibers of a core-forming material containing an absorbent material. As the absorbent material, an absorbent material that is conventionally used to form this type of absorbent core can be used without particular limitation. For example, it is possible to use wood pulp, hydrophilic fibers such as synthetic fibers that have been treated with a hydrophilizing agent, and absorbent polymer particles. That is to say, the absorbent core 40 may be made of a fiber stack made of hydrophilic fibers, or absorbent polymer particles that are supported on the fiber stack. As the core-wrap sheet 41, a liquid permeable sheet material can be used, and examples thereof include paper, non-woven fabric, and the like.

The intermediate sheet 5 is a sheet that is different from the absorbent member 4 and is interposed between the topsheet 2 and the absorbent member 4, and is a sheet that is also called a sublayer or a second sheet. The intermediate sheet 5 is rectangular in a plan view as shown in Fig. 1. The intermediate sheet 5 has its lengthwise direction matching the longitudinal direction X, and preferably covers 30 to 70%, and more preferably 40 to 60% of the skin-facing surface of the absorbent member 4. In the diaper 1, the intermediate sheet 5 is arranged in the crotch portion 1M. The intermediate sheet 5 and each of the topsheet 2 and the absorbent member 4 (the core-wrap sheet 41) are entirely or partially joined by a known joining means such as an adhesive. The intermediate sheet 5 will be described later in detail.

Side sheets 6 are respectively provided on the right and left sides in the longitudinal direction X of the topsheet 2. Each of the side sheets 6 includes an inner edge portion that extends in the longitudinal direction X, and an outer edge portion that is located on the outer side in the lateral direction Y with respect to the inner edge portion, and extends in the longitudinal direction X. In a plan view as shown in Fig. 1, the inner edge portions overlap the absorbent member 4, and, as shown in Fig. 2, the outer edge portions extend outward in the lateral direction Y from side edges of the absorbent member 4 that extends in the longitudinal direction X and are joined to the backsheet 3. Between the side sheet 6 and the backsheet 3 in each of the right and left leg portions that are to be placed around the legs of the wearer, an elastic member 60 in the form of strings is fixed in a stretched state so as to extend in the longitudinal direction X. With this configuration, in the leg portions of the diaper 1 when worn, a pair of leg cuffs are formed due to the elastic members 60 contracting. Also, in the inner edge portion of each side sheet 6, an elastic member 61 in the form of strings is fixed in a stretched state so as to extend in the longitudinal direction X. With this configuration, when the diaper 1 is worn, due to contraction of the elastic members 61, at least in the crotch portion 1M, the side sheets 6 each form a leakage-preventing cuff as a result of the inner edge portion standing toward the skin of the wearer from the joint portion between the side sheets 6 and the backsheet 3. The leakage-preventing cuffs can prevent a waste liquid such as urine from flowing toward the outside in the lateral direction Y, that is, so-called side leakage. The topsheet 2, the backsheet 3, the absorbent member 4, the intermediate sheet 5, the side sheets 6, and the elastic members 60 and 61 are joined to each other by a known joining means such as a hot melt adhesive or the like.

The diaper 1 is a so-called open type disposable diaper, and, as shown in Fig. 1, two pieces of fastening tape 7 are respectively provided at two side edge portions that extend in the longitudinal direction X of the rear portion 1R of the diaper 1. An unshown fastening portion that is a male member of a mechanical hook-and-loop fastener is attached to each fastening tape 7. Also, a target region 8 that is a female member of the mechanical hook-and-loop fastener is formed in the non-skin-facing surface of the front portion 1F of the diaper 1. The target region 8 is formed by joining and fixing the female member of the mechanical hook-and-loop fastener to the non-skin-facing surface of the backsheet 3 that forms the non-skin-facing surface of the front portion 1F using a known joining means such as, for example, an adhesive or through heat sealing, and is configured to removably receive the fastening portions of the pieces of fastening tape 7.

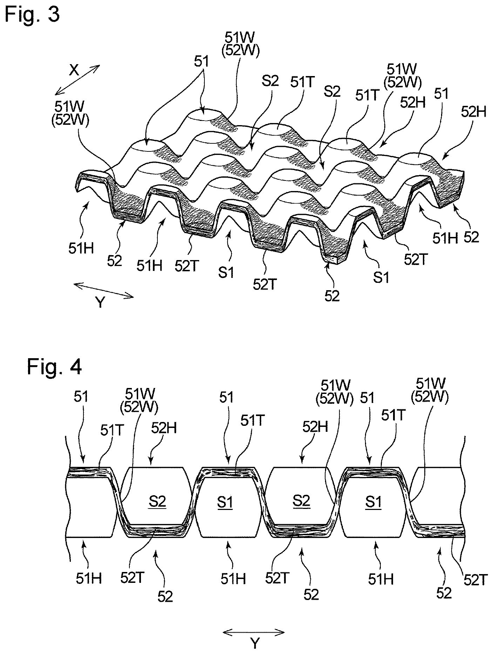

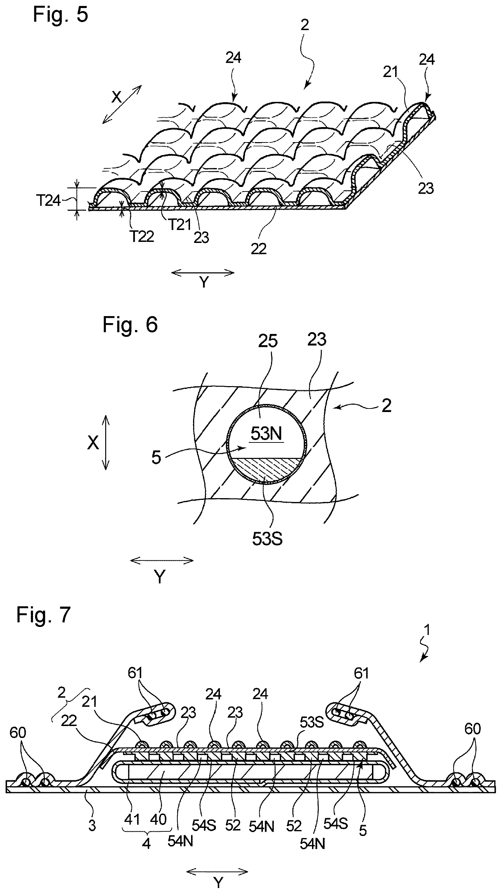

Next, the intermediate sheet 5 described above will be described in detail with reference to Figs. 3 and 4 in addition to Figs. 1 and 2. Fig. 3 shows a perspective view of the intermediate sheet 5, and Fig. 4 shows a cross-sectional view of the intermediate sheet 5. Furthermore, the topsheet 2 described above will be described in detail with reference to Fig. 5 in addition to Figs. 1 and 2. Fig. 5 shows a perspective view of the topsheet 2. First, the intermediate sheet 5 will be described.

As shown in Fig. 3, the intermediate sheet 5 has a plurality of skin-side projections 51 projecting toward the skin-facing surface side and having internal spaces S1, and skin-side recesses positioned between the plurality of skin-side projections 51, and further has a plurality of non-skin-side projections 52 projecting toward the non-skin-facing surface side and having internal spaces S2, and non-skin-side recesses positioned between the plurality of non-skin-side projections 52. In the configuration shown in Fig. 3, the projections as viewed from the topsheet 2 side that is the skin-facing surface side are the skin-side projections 51, and the skin-side recesses are the non-skin-side projections 52. Conversely, the projections as viewed from the absorbent member 4 side that is the non-skin-facing surface side are the non-skin-side projections 52, and the non-skin-side recesses are the skin-side projections 51. Accordingly, the skin-side projections 51 and the non-skin-side projections 52 are partially shared. The shared portions correspond to later-described wall portions 51W having ring-like structures, and later-described wall portions 52W having ring-like structures. In this manner, the intermediate sheet 5 is an uneven sheet. Note that the internal spaces S1 and the internal spaces S2 do not include gaps between constituent fibers forming the intermediate sheet 5, and, specifically, do not include very small gaps with a fiber-to-fiber distance of approximately 0.01 to 0.2 mm.

There is no particular limitation on the arrangement of the skin-side projections 51 and the non-skin-side projections 52, but, in the diaper 1, in a plan view of the intermediate sheet 5 as shown in Fig. 1, the skin-side projections 51 and the non-skin-side projections 52 are alternately arranged in a successive manner in two different directions that intersect each other. In this case, the two different directions are preferably such that a first direction in the plane, and a second direction intersecting the first direction intersect each other at an angle of 30 to 90 degrees, and, for example, they intersect each other at 90 degrees in the diaper 1. That is to say, in the diaper 1, one of the two directions is the lateral direction Y, and the other of the two directions is the longitudinal direction X. Accordingly, the skin-side projections 51 and the non-skin-side projections 52 are alternately arranged in a successive manner along the longitudinal direction X and the lateral direction Y.

As shown in Fig. 2, the uneven intermediate sheet 5 is arranged between the topsheet 2 and the absorbent member 4. Accordingly, top portions 51T of the skin-side projections 51 are in contact with the topsheet 2 on the skin-facing surface of the intermediate sheet 5, and top portions 52T of the non-skin-side projections 52 are in contact with the absorbent member 4, which is a member adjacent to the non-skin-facing surface of the intermediate sheet 5, on the non-skin-facing surface of the intermediate sheet 5. The skin-facing surface of the intermediate sheet 5 has a plurality of skin-side contact portions 53S that are in contact with the topsheet 2 at the skin-side projections 51, and skin-side non-contact portions 53N that are not in contact with the topsheet 2. In the diaper 1, on the skin-facing surface of the intermediate sheet 5, the skin-side contact portions 53S are separate from each other in both of the longitudinal direction X and the lateral direction Y, and each of the skin-side contact portions 53S is surrounded by the skin-side non-contact portions 53N. Furthermore, the non-skin-facing surface of the intermediate sheet 5 has a plurality of contact portions 54S that are in contact with the absorbent member 4 at the non-skin-side projections 52, and non-contact portions 54N that are not in contact with the absorbent member 4. In the diaper 1, on the non-skin-facing surface of the intermediate sheet 5, the contact portions 54S are separate from each other in both of the longitudinal direction X and the lateral direction Y, and each of the contact portions 54S is surrounded by the non-contact portions 54N. In this manner, the contact portions 54S or the skin-side contact portions 53S are separate from each other in two directions, and thus a continuous space in which later-described internal spaces S1 are continuously formed or a continuous space in which later-described internal spaces S2 are continuously formed is likely to extend in the planar direction, and thus the dispersibility of loose stools is improved. Note that the skin-side non-contact portions 53N and the non-contact portions 54N do not include gaps between constituent fibers forming the intermediate sheet 5, and, specifically, do not include very small gaps with a fiber-to-fiber distance of approximately 0.01 to 0.2 mm.

As shown in Fig. 2, the skin-facing surface of the intermediate sheet 5 has the skin-side non-contact portions 53N that are recessed so as to project toward the non-skin-facing surface side, and that is not in contact with the topsheet 2. Specifically, at the contact portions 54S on the non-skin-facing surface, the non-skin-side projections 52 are in contact with the absorbent member 4, and the skin-facing surface of the intermediate sheet 5 that is positioned at the contact portions 54S is recessed so as to project toward the non-skin-facing surface side, and thus the skin-side non-contact portions 53N are formed. In this manner, the skin-side non-contact portions 53N are arranged at positions corresponding to the contact portions 54S. The internal portions of the non-skin-side projections 52 are not filled with constituent fibers and form hollow spaces. Since the internal portions of the non-skin-side projections 52 form hollow spaces, loose stools that have shifted from the topsheet 2 on the skin-facing surface side of the intermediate sheet 5 can be stocked in these internal portions without being dispersed. In a similar manner, at the skin-side contact portions 53S on the skin-facing surface, the skin-side projections 51 are in contact with the topsheet 2, and the non-skin-facing surface of the intermediate sheet 5 that is positioned at the skin-side contact portions 53S is recessed so as to project toward the skin-facing surface side, and thus the skin-side contact portions 53S are formed. In this manner, the skin-side contact portions 53S are arranged at positions corresponding to the non-contact portions 54N on the non-skin side. The internal portions of the skin-side projections 51 are not filled with constituent fibers, and form hollow internal spaces.

In a plan view of the intermediate sheet 5 from the absorbent member 4 side, which is the non-skin-facing surface side, the adjacent distance between adjacent contact portions 54S that are the closest to each other is preferably 0.5 mm or more, and more preferably 1 mm or more, is preferably 10 mm or less, and more preferably 8 mm or less, and, specifically, is preferably from 0.5 to 10 mm, and more preferably from 1 to 8 mm, from the viewpoint of improving the dispersibility of loose stools that have shifted.

In a plan view of the intermediate sheet 5 from the topsheet 2 side, which is the skin-facing surface side, the adjacent distance between adjacent skin-side contact portions 53S that are the closest to each other is preferably 0.5 mm or more, and more preferably 1 mm or more, is preferably 10 mm or less, and more preferably 8 mm or less, and, specifically, is preferably from 0.5 to 10 mm, and more preferably from 1 to 8 mm, from the viewpoint of stocking loose stools that have shifted. The adjacent distance between is measured using the following measuring method.

### Method for Measuring Adjacent Distance

The adjacent distance between adjacent contact portions 54S on the non-skin-facing surface of the intermediate sheet 5 or the adjacent distance between adjacent skin-side contact portions 53S on the skin-facing surface can be measured using the following method.

First, a cut sample with a size of 50 mm (CD direction) × 50 mm (MD direction) is cut out from the intermediate sheet 5. The cut sample is placed without applying pressure such that the side that is to be measured (e.g., the non-skin-facing surface on the absorbent member 4 side) is oriented upward, a transparent acrylic plate with a weight of 50 g is placed from above, and a weight of 700 g is further installed on the acrylic plate. In a state in which a load of 30 gf/cm² is applied, the surface shape of the cut sample is measured in a range of 40 mm (CD direction) × 40 mm (MD direction) using a high-precision shape measurement system KS-1100 manufactured by Keyence at a measurement pitch of 50 µm and a movement speed of 10 cm/s, and an image is captured. Next, the captured image is analyzed using a shape analysis application KS-Analyzer manufactured by Keyence, a portion that extends from a position with the largest thickness to a portion with a thickness smaller by 500 µm than the largest thickness is extracted and subjected to binarization processing, and thus an image of a portion that is in contact with a member (the absorbent member 4) adjacent to the non-skin-facing surface is obtained. This image is captured by an Image-Pro Plus (manufactured by Nippon Roper K.K.), the black and white contrast is set to 100 by emphasizing the contrast, and noise is removed through filtering processing (median, 5 × 5 is performed five times). Subsequently, a line connecting centers of gravity of adjacent contact portions 54S is drawn. The center of gravity of a contact portion 54S is obtained as follows: a perpendicular line that is perpendicular to the Feret diameter of one contact portion 54S is drawn so as to pass through the center of the Feret diameter length, and the center between two points at which the perpendicular line intersects the contour of the contact portion is taken as the center of gravity. In this manner, a line connecting centers of gravity of contact portions that are the closest to each other is drawn, the distance of the non-contact portion 54N on that line is measured, and the thus obtained measurement value is taken as the adjacent distance between the contact portions 54S. Note that the adjacent distance between the skin-side contact portions 53S is measured as follows: the cut sample is placed such that the side that is to be measured (e.g., the skin-facing surface on the topsheet 2 side) is oriented upward, and measurement is performed in a similar way to that of the adjacent distance between the contact portions 54S. The load 30 gf/cm² is assumed to be pressure (pressure that is to be withstood) that will be applied to the rear portion of the diaper 1 when a baby that is a few months old and wearing the diaper is sleeping lying face up.

When viewed in a cross-section of the diaper 1, a height HI (see Fig. 2) of the internal spaces S1 between the skin-side projections 51 and the absorbent member 4 is preferably 0.5 mm or more, and more preferably 1 mm or more, is preferably 20 mm or less, and more preferably 10 mm or less, and, specifically, is preferably from 0.5 to 20 mm, and more preferably from 1 to 10 mm, from the viewpoint of improving the dispersibility of loose stools that have shifted and improving the air permeability when worn.

Furthermore, when viewed in a cross-section of the diaper 1, a height H2 (see Fig. 2) of the internal spaces S2 between the non-skin-side projections 52 and the topsheet 2 is preferably 0.5 mm or more, and more preferably 1 mm or more, is preferably 20 mm or less, and more preferably 10 mm or less, and, specifically, is preferably from 0.5 to 20 mm, and more preferably from 1 to 10 mm, from the viewpoint of improving the dispersibility of loose stools that have shifted and improving the air permeability when worn. The heights H1 and H2 are measured under no load by observing a cross-section in the thickness direction of the diaper 1 using a microscope.

As shown in Figs. 3 and 4, the skin-side projections 51 respectively have the wall portions 51W having ring-like structures and located between the top portions 51T and opening portions 51H of the internal spaces S1. Furthermore, the non-skin-side projections 52 respectively have the wall portions 52W having ring-like structures and located between the top portions 52T and opening portions 52H of the internal spaces S2. There is no limitation on the projecting shape of the skin-side projections 51 and the non-skin-side projections 52, and examples thereof include shapes of cones such as a circular cone, a truncated cone, a pyramid, a truncated pyramid, and an oblique cone. In the diaper 1, the skin-side projections 51 and the non-skin-side projections 52 are formed in the shape of truncated pyramids in which the top portions 51T and the top portions 52T are flat.

In a plan view from the skin-facing surface side of the intermediate sheet 5, the wall portions 51W of the skin-side projections 51 have ring-like structures respectively about the top portions 51T of the skin-side projections 51. Furthermore, in a plan view from the non-skin-facing surface side of the intermediate sheet 5, the wall portions 52W of the non-skin-side projections 52 have ring-like structures respectively about the top portions 52T of the non-skin-side projections 52. As shown in Figs. 3 and 4, the wall portions 51W of the skin-side projections 51 share the same regions as the wall portions 52W of the non-skin-side projections 52. In this example, there is no particular limitation on the "ring-like" structure, as long as it has an endless continuous shape in a plan view of the intermediate sheet 5, and examples thereof in a plan view of the intermediate sheet 5 include any shapes such as a circle, an ellipse, a rectangle, and a polygon, and the like, but it is preferable to have the shape of a circular cylinder, an elliptic cylinder, a truncated cone, or a truncated elliptic, from the viewpoint of maintaining the internal spaces even when pressure is applied thereto.

Furthermore, when taking the "ring-like" structure as a three-dimensional shape, examples thereof include any ring-like structures having the shapes of a circular cylinder, an oblique circular cylinder, an elliptic cylinder, a truncated cone, a truncated oblique cone, a truncated elliptic cone, a truncated quadrangular pyramid, a truncated oblique quadrangular pyramid, and the like, but it is preferable to have the shape of a circular cylinder, an elliptic cylinder, a truncated cone, or a truncated elliptic cone, from the viewpoint of maintaining the internal spaces even when pressure is applied thereto.

As described above, the intermediate sheet 5 is formed from an uneven nonwoven fabric in which the plurality of skin-side projections 51 and the non-skin-side projections 52 are arranged in the longitudinal direction X and the lateral direction Y as shown in Fig. 3. Furthermore, as shown in Fig. 2, the intermediate sheet 5 has the skin-side contact portions 53S that are in contact with the topsheet 2 adjacent to the intermediate sheet 5 at the skin-side projections 51, and forms the internal spaces S2 between the skin-side projections 51 and the topsheet 2. As shown in Fig. 4, adjacent internal spaces S2 are interconnected and form a continuous space. In a similar manner, as shown in Fig. 2, the intermediate sheet 5 has the contact portions 54S that are in contact with the absorbent member 4 adjacent to the intermediate sheet 5 at the non-skin-side projections 52, and forms the internal spaces S1 between the non-skin-side projections 52 and the absorbent member 4. As shown in Fig. 4, adjacent internal spaces S1 are interconnected and form a continuous space.

From the viewpoint of making it possible to effectively absorb loose stools, improving the dispersibility of loose stools that have been absorbed, suppressing return of loose stools to the topsheet 2, and suppressing attachment of loose stools to the wearer's skin, when the intermediate sheet 5 is pressed from the absorbent member 4 side in the thickness direction at a pressure of 30 gf/cm², the proportion of the total area of contact faces T54, on which the plurality of contact portions 54S located on a pressed face Ta that has been pressed are in contact with the absorbent member 4, with respect to the pressed face Ta ((the total area of the contact faces T54 / the pressed face Ta) × 100) (a non-skin-side contact area percentage R54) is 80% or less, preferably 75% or less, and more preferably 70% or less, is preferably 20% or more, and more preferably 25% or more, and, specifically, is preferably from 20 to 75%, and more preferably from 25 to 70%. In this example, the pressed face Ta means a certain face in a predetermined range that is pressed at a pressure of 30 gf/cm², and, in a later-described method for measuring the contact area percentage, it is a face in a range that is pressed by an acrylic plate that is a non-deformable member. In other words, it is also an area of a sample that is pressed by an acrylic plate. Note that the pressure 30 gf/cm² is assumed to be pressure (pressure that is to be withstood) that will be applied to the rear portion of the diaper 1 when a baby that is a few months old and wearing the diaper is sleeping lying face up. The non-skin-side contact area percentage R54 is measured using the following measuring method.

From the viewpoint of making it possible to effectively absorb loose stools and to easily stock loose stools that have been absorbed, suppressing return of loose stools to the topsheet 2, and suppressing attachment of loose stools to the wearer's skin, when the intermediate sheet 5 is pressed from the topsheet 2 side in the thickness direction at a pressure of 30 gf/cm², the proportion of the total area of contact faces T53, in which the plurality of skin-side contact portions 53S located on a pressed face Ta that has been pressed are in contact with the topsheet 2, with respect to the pressed face Ta ((the total area of the contact faces T53 / the pressed face Ta) × 100) (a skin-side contact area percentage R53) is preferably larger than the proportion of the total area of the contact faces T54 on which the contact portions 54S are in contact with the absorbent member 4 (the non-skin-side contact area percentage R54). Specifically, the ratio of the skin-side contact area percentage R53 with respect to the non-skin-side contact area percentage R54 is preferably 1.1 or more, and more preferably 1.5 or more, is preferably 10 or less, and more preferably 7 or less, and, specifically, is preferably from 1.1 to 10, and more preferably from 1.5 to 7.

From similar viewpoints, the skin-side contact area percentage R53 is preferably 90% or less, and more preferably 85% or less, is preferably 20% or more, and more preferably 30% or more, and, specifically, is preferably from 20 to 90%, and more preferably from 30 to 85%. The skin-side contact area percentage R53 is measured using the following measuring method.

### Method for Measuring Contact Area Percentage

The contact area between the intermediate sheet 5 and a member (the topsheet 2) adjacent to the skin-facing surface of the intermediate sheet 5 or a member (the absorbent member 4) adjacent to the non-skin-facing surface can be measured using the following method.

First, a cut sample with a size of 50 mm (CD direction) × 50 mm (MD direction) is cut out from the intermediate sheet 5. The cut sample is placed without applying pressure such that the side that is to be measured (e.g., the non-skin-facing surface on the absorbent member 4 side) is oriented upward, a transparent acrylic plate with a weight of 50 g is placed from above, and a weight of 700 g is further installed on the acrylic plate. In a state in which a load of 30 gf/cm² is applied, the surface shape of the cut sample is measured in a range of 40 mm (CD direction) × 40 mm (MD direction) using a high-precision shape measurement system KS-1100 manufactured by Keyence at a measurement pitch of 50 µm and a movement speed of 10 cm/s, and an image is captured. Next, the captured image is analyzed using a shape analysis application KS-Analyzer manufactured by Keyence, a portion that extends from a position with the largest thickness to a portion with a thickness smaller by 500 µm than the largest thickness is extracted and subjected to binarization processing, and thus an image of a portion that is in contact with a member (the absorbent member 4) adjacent to the non-skin-facing surface is obtained. This image is captured by an Image-Pro Plus (manufactured by Nippon Roper K.K.), the black and white contrast is set to 100 by emphasizing the contrast, noise is removed through filtering processing (median, 5 × 5 is performed five times), and counting processing is performed to measure the black and white areas. Subsequently, the total black area, which is the total area of the contact faces T54 on which the plurality of contact portions 54S are in contact with the absorbent member 4, is divided by the measurement range area (40 mm (CD direction) × 40 mm (MD direction)), which is the total of the black and white areas, so that the non-skin-side contact area percentage R54 is calculated. Note that the skin-side contact area percentage R53 is measured as follows: the cut sample is placed such that the side that is to be measured (e.g., the skin-facing surface on the topsheet 2 side) is oriented upward, and measurement is performed in a similar way to that of the non-skin-side contact area percentage R54.

Furthermore, the basis weight of the intermediate sheet 5 may be selected as appropriate according to the application, and, when a sheet is used as an intermediate sheet for a disposable diaper, the average value of the entire sheet is preferably from 10 to 80 g/m², and more preferably from 15 to 70 g/m².

The intermediate sheet 5 described above can be produced, for example, using an air-through nonwoven fabric or the like having a monolayer structure or a multilayer structure in which multiple layers are layered as a raw material nonwoven fabric, by placing the air-through nonwoven fabric or the like on a support member having a large number of protrusions, and applying warm air to the air-through nonwoven fabric or the like on the support member, thereby shaping the through nonwoven fabric or the like into an uneven shape. Note that the shape of the protrusions is a shape corresponding to the projecting shape of the skin-side projections 51 and the non-skin-side projections 52, and examples thereof include shapes of cones such as a circular cone, a truncated cone, a pyramid, a truncated pyramid, and an oblique cone. Aspects of the method for producing the intermediate sheet 5 not specifically described may be similar to those of the methods described, for example, in JP 2013-133574A, JP 2012-149370A, JP 2012-149371A, or the like.

Next, as the topsheet 2, a flat nonwoven fabric and the like conventionally used as a topsheet to form this type of absorbent article can be used without particular limitation. It is more effective to use a nonwoven fabric or the like with a specific uneven shape according to the application of the above-described characteristic configuration of the intermediate sheet 5. The uneven topsheet 2 may be formed from one sheet member, but in the diaper 1 it is formed from a layered member of a first sheet 21 and a second sheet 22 as shown in Figs. 2 and 5. The first sheet 21 is located on the skin-facing surface side, and the second sheet 22 is located on the non-skin-facing surface side. That is to say, the first sheet 21 is located on the wearer's body side when the diaper 1 is worn. The topsheet 2 has a plurality of joined regions 23 formed by partially joining the first sheet 21 and the second sheet 22. The first sheet 21 projects in a direction away from the second sheet 22 in regions other than the joined regions 23, the regions being surrounded by the joined regions 23, and forms a plurality of projections 24 projecting toward the wearer's skin-side. The second sheet 22 of the topsheet 2 is substantially flat, and the first sheet 21 has an uneven shape with a large undulation level, and thus the plurality of projections 24 formed from the first sheet 21 are formed. In this manner, the topsheet 2 has such an uneven shape, and thus, dispersion of loose stools in the planar direction when the diaper 1 is worn can be suppressed. Furthermore, since the second sheet 22 of the topsheet 2 is substantially flat, the wearer's body pressure when the diaper 1 is worn can be dispersed over a wide range of the intermediate sheet 5. In particular, the intermediate sheet 5 has projections that are formed in the shape of truncated pyramids in which the top portions 51T and the top portions 52T are flat, and thus the above-described effect can be more reliably achieved.

It is sufficient that the plurality of projections 24 are arranged at least in the crotch portion 1M, but in the diaper 1 they are arranged over the entire range from the front portion 1F to the rear portion 1R. The projections 24 of the topsheet 2 preferably have the following configuration.

A height T24 (see Fig. 5) of the projections 24 is preferably 0.3 mm or more, and more preferably 0.5 mm or more, is preferably 3 mm or less, and more preferably 2.5 mm or less, and, more specifically, is preferably from 0.3 to 3 mm, and more preferably from0.5 to 2.5 mm.

A thickness T21 (see Fig. 5) of the first sheet 21 for forming the projections 24 is preferably 0.1 mm or more, and more preferably 0.2 mm or more, is preferably 3 mm or less, and more preferably 2 mm or less, and, more specifically, is preferably from 0.1 to 3 mm, and more preferably from 0.2 to 2 mm.

A thickness T22 (see Fig. 5) of the second sheet 22 is preferably 0.1 mm or more, and more preferably 0.2 mm or more, is preferably 3 mm or less, and more preferably 2 mm or less, and, more specifically, is preferably from 0.1 to 3 mm, and more preferably from 0.2 to 2 mm.

Furthermore, the number of projections 24 per area of 10 cm² on the uneven topsheet 2 is preferably from 10 to 150, and more preferably from 30 to 120.

As sheet materials for forming the first sheet 21 and the second sheet 22, various sheet materials known as a material for forming a topsheet in absorbent articles such as disposable diapers or sanitary napkins can be used, but it is preferable to use a nonwoven fabric from the viewpoint of improving the liquid permeability and the feel on the skin. As constituent fibers of a nonwoven fabric, heat fusible fibers are preferably used. A nonwoven fabric may contain, in addition to heat fusible fibers, fibers that are not heat fusible (e.g., cotton fibers, etc.) as constituent fibers. The basis weight of the first sheet 21 and the second sheet 22 may be selected as appropriate according to the application, and, when a sheet is used as the topsheet 2 for a disposable diaper, the basis weight is preferably 5 g/m² or more, and more preferably 10 g/m² or more, is preferably 40 g/m² or less, and more preferably 30 g/m² or less, and is preferably from 5 to 40 g/m², and more preferably from 10 to 30 g/m².

The plurality of joined regions 23 can be formed using various methods. If the first sheet 21 and the second sheet 22 contain heat fusible fibers, it is possible to use fusion-bonding using heat, ultrasonic waves, high frequency, or the like. Regardless of the material for the first sheet 21 and the second sheet 22, it is possible to use bonding using an adhesive. At the joined regions 23, the first sheet 21 and the second sheet 22 are pressed in one piece, and the sheets 21 and 22 have a density higher than that in the other portions. Preferably, the sheets 21 and 22 are bonded to each other though thermal fusion-bonding in which a constituent resin of one or both of the sheets are melted and then cured.

As shown in Figs. 2 and 6, the joined regions 23 of the topsheet 2 have holes 25 that extend through the joined regions 23. Since the topsheet 2 has an uneven shape, loose stools are likely to remain in recesses between the projections 24. Furthermore, since the holes 25 are formed at the joined regions 23 at the recess bottoms, the remaining loose stools are likely to shift to the intermediate sheet 5. From the viewpoint of causing the skin-side non-contact portions 53N, in which the skin-facing surface is recessed, to stock loose stools that have shifted to the intermediate sheet 5, the intermediate sheet 5 is preferably arranged such that the holes 25 of the topsheet 2 and the skin-side non-contact portions 53N on the skin-facing surface of the intermediate sheet 5 overlap each other in the thickness direction. The holes 25 and the skin-side non-contact portions 53N overlap each other over an area of preferably 20% or more, and more preferably 30% or more. It is preferable that the percentage of holes 25 that overlap, over an area of 20% or more, the skin-side non-contact portions 53N is larger with respect to all holes 25, but, if the percentage is 30% or more with respect to all holes 25, the above-described effect can be more reliably achieved. The area over which the holes 25 and the skin-side non-contact portions 53N overlap each other can be measured as follows.

### Method for Measuring Area over which Holes 25 and Skin-Side Non-Contact Portions 53N Overlap each other

A transparent acrylic plate with a weight of 50 g is placed from above on the topsheet 2, and an image viewed from above with a load of 30 gf/cm² applied thereto is captured. Then, a hole area S25 of the holes 25 of the topsheet 2 and an area S53N of the skin-side non-contact portions 53N, of the intermediate sheet 5, that overlap the holes 25 are measured from the captured image viewed from above, the area percentage ((S53N/S25) × 100) is calculated from the measured values, and this value is obtained as the area over which the holes 25 and the skin-side non-contact portions 53N overlap each other. Note that, in other words, the area S53N of the skin-side non-contact portions 53N that overlap the holes 25 is a value obtained by subtracting the area of the skin-side contact portions 53S that overlap the holes 25 from the hole area S25 of the holes 25.

In the topsheet 2 described above, for example, the first sheet 21 is shaped into an uneven shape by guiding the first sheet 21 into a portion in which a first roller whose circumferential face has an uneven shape meshes with a second roller whose circumferential face has an uneven shape that conforms to the uneven shape of the first roller. While the first sheet 21 shaped into an uneven shape is held with the uneven shape being maintained on the circumferential face of the first roller through suction, the second sheet 22 is placed over the first sheet 21 shaped into an uneven shape, and the second sheet 22 is joined to the first sheet 21 at positions on the projections of the first roller, so that the joined regions 23 are formed, and, at the same time, the holes 25 are formed at the joined regions 23. Accordingly, the topsheet 2 can be produced. Aspects of the method for producing the topsheet 2 not specifically described may be similar to the method for producing a topsheet described in JP 2004-174234A or the method for producing a composite sheet described in JP 2008-106420A.

The absorbent article according to the present invention may be, for example, the diaper 1 according to an embodiment shown in Fig. 7, as well as the diaper 1 shown in Figs. 1 and 2. The diaper 1 shown in Fig. 7 will be described mainly focusing on constituent elements different from those in the diaper 1 shown in Figs. 1 and 2, and the same constituent elements are denoted by the same reference numerals and a description thereof has been omitted. The description of constituent elements of the diaper 1 shown in Figs. 1 and 2 may be applied as appropriate to those not specifically described.

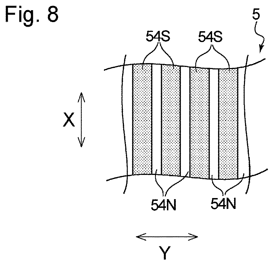

In the diaper 1 shown in Fig. 7, the intermediate sheet 5 is formed such that the skin-facing surface is flat without having the skin-side projections 51 projecting toward the skin-facing surface side, but has the non-skin-side projections 52 projecting toward the non-skin-facing surface side. The intermediate sheet 5 shown in Fig. 7 is formed from one nonwoven fabric. Since the diaper 1 shown in Fig. 7 is formed such that the skin-facing surface of the intermediate sheet 5 does not have the skin-side projections 51, the entire skin-facing surface of the intermediate sheet 5 is in contact with the topsheet 2, and the skin-side contact portions 53S are formed on the entire skin-facing surface of the intermediate sheet 5. Furthermore, in the diaper 1 shown in Fig. 7, the internal portions of the non-skin-side projections 52 formed on the non-skin-facing surface of the intermediate sheet 5 are filled with constituent fibers, and are formed solid. The contact portions 54S on the non-skin-facing surface are formed by bringing the top portions of the solid non-skin-side projections 52 into contact with the absorbent member 4. Note that the non-skin-facing surface between the solid non-skin-side projections 52 is recessed so as to project toward the topsheet 2 side, and thus the non-contact portions 54N that are not in contact with the absorbent member 4 are formed. In the diaper 1 shown in Fig. 7, in a plan view of the intermediate sheet 5 from the absorbent member 4 side as shown in Fig. 8, each of the contact portions 54S and the non-contact portions 54N on the non-skin-facing surface of the intermediate sheet 5 is continuously formed along the longitudinal direction X, and the contact portions 54S and the non-contact portions 54N are alternately arranged along the lateral direction Y. In this manner, the plurality of contact portions 54S are separate from each other in the lateral direction Y that is one direction. In the diaper 1 shown in Fig. 7, each of the non-contact portions 54N is continuously formed along the longitudinal direction X, and thus loose stools that have shifted to the intermediate sheet 5 can be dispersed in the longitudinal direction X, return of loose stools to the topsheet 2 can be suppressed, and loose stools are unlikely to be attached to the wearer's skin.

The intermediate sheet 5 shown in Fig. 7 can be produced, for example, by pressing one nonwoven fabric containing heat fusible fibers using a heated pressing member having the shape that conforms to the non-contact portions 54N. The portions not pressed by the pressing member form projections, and the top portions of the projections are brought into contact with the absorbent member 4 and form the skin-side contact portions 53S.

The absorbent article according to the present invention was described above based on preferred embodiments thereof, but the absorbent article according to the present invention is not limited to the foregoing embodiments, and various changes may be made.

For example, the absorbent article according to the present invention is not limited to the above-described open-type disposable diaper, and may also be a pull-on disposable diaper.

### Examples

Hereinafter, the present invention will be described in more detail by way of examples, but the invention is not limited to these examples.

### Example 1

As an intermediate sheet, an uneven intermediate sheet with a basis weight of 40 g/m² was prepared in which the skin-side projections 51 and the non-skin-side projections 52 were alternately arranged in a successive manner along the longitudinal direction X and the lateral direction Y. The prepared intermediate sheet had an adjacent distance between adjacent contact portions 54S that were the closest to each other of 5 mm, a height HI (see Fig. 2) of the internal spaces S1 between the skin-side projections 51 and the absorbent member 4 of 2.3 mm, and a non-skin-side contact area percentage R54 at a pressure of 30 gf/cm² of 73%.

Next, as an open-type disposable diaper, a Merries tape-type diaper, S size (manufactured in 2017, registered trademark) manufactured by Kao Corporation was prepared. A topsheet of the prepared diaper was peeled away by curing a hot melt bonding the materials through cold spraying. Then, the prepared uneven intermediate sheet was arranged such that the skin-side projections 51 were located on the topsheet side and the non-skin-side projections 52 were located on the absorbent member side, the peeled topsheet was arranged on and fixed to the intermediate sheet, so that a disposable diaper of Example 1 as shown in Figs. 1 and 2 was produced. Note that the peeled topsheet was an uneven sheet without having holes.

The adjacent distance and the non-skin-side contact area percentage R54 were measured using a method similar to the above-described measuring methods.

### Example 2

A disposable diaper of Example 2 was produced in a similar way to that of the disposable diaper of Example 1, except that an uneven air-through nonwoven fabric with a basis weight of 40 g/m² was used as an intermediate sheet. The intermediate sheet had an adjacent distance between adjacent contact portions 54S that were the closest to each other of 4 mm, a height H1 (see Fig. 2) of the internal spaces S1 between the skin-side projections 51 and the absorbent member 4 of 3.5 mm, and a non-skin-side contact area percentage R54 at a pressure of 30 gf/cm² of 43%.

### Example 3

A disposable diaper of Example 3 was produced in a similar way to that of the disposable diaper of Example 1, except that an uneven air-through nonwoven fabric with a basis weight of 40 g/m² was used as an intermediate sheet. The intermediate sheet had an adjacent distance between adjacent contact portions 54S that were the closest to each other of 5 mm, a height HI (see Fig. 2) of the internal spaces S1 between the skin-side projections 51 and the absorbent member 4 of 2.3 mm, and a non-skin-side contact area percentage R54 at a pressure of 30 gf/cm² of 40%.

### Example 4

A disposable diaper of Example 4 was produced in a similar way to that of the disposable diaper of Example 1, except that an uneven air-through nonwoven fabric with a basis weight of 40 g/m² was used as an intermediate sheet. The intermediate sheet had an adjacent distance between adjacent contact portions 54S that were the closest to each other of 2 mm, a height HI (see Fig. 2) of the internal spaces S1 between the skin-side projections 51 and the absorbent member 4 of 1.5 mm, and a non-skin-side contact area percentage R54 at a pressure of 30 gf/cm² of 28%.

### Example 5

A disposable diaper of Example 5 was produced in a similar way to that of the disposable diaper of Example 1, in which a topsheet obtained by forming holes through recesses of the uneven topsheet used in Example 1 was used as a topsheet and an intermediate sheet used in Example 1 was used as an intermediate sheet.

### Example 6

A disposable diaper of Example 6 was produced in a similar way to that of the disposable diaper of Example 1, except that the topsheet having holes used in Example 5 was used as a topsheet and the intermediate sheet used in Example 2 was used as an intermediate sheet.

### Example 7

A disposable diaper of Example 7 was produced in a similar way to that of the disposable diaper of Example 1, except that the topsheet having holes used in Example 5 was used as a topsheet and the intermediate sheet used in Example 3 was used as an intermediate sheet.

### Example 8

A disposable diaper of Example 8 was produced in a similar way to that of the disposable diaper of Example 1, except that the topsheet having holes used in Example 5 was used as a topsheet and the intermediate sheet used in Example 4 was used as an intermediate sheet.

### Comparative Example 1

A Merries tape-type diaper, S size (manufactured in 2017, registered trademark) manufactured by Kao Corporation without having an intermediate sheet was used as a diaper of Comparative Example 1.

### Comparative Example 2

A disposable diaper of Comparative Example 2 was produced in a similar way to that of the disposable diaper of Example 1, except that a flat air-through nonwoven fabric not having an uneven shape with a basis weight of 40 g/m² was used as an intermediate sheet.

### Comparative Example 3

A disposable diaper of Comparative Example 3 was produced in a similar way to that of the disposable diaper of Example 1, except that an uneven air-through nonwoven fabric with a basis weight of 40 g/m² was used as an intermediate sheet. The intermediate sheet had an adjacent distance between adjacent contact portions 54S that were the closest to each other of 2 mm, a height HI (see Fig. 2) of the internal spaces S1 between the skin-side projections 51 and the absorbent member 4 of 1 mm, and a non-skin-side contact area percentage R54 at a pressure of 30 gf/cm² of 88%.

### Comparative Example 4

A Merries tape-type diaper, S size (manufactured in 2017, registered trademark) manufactured by Kao Corporation without having an intermediate sheet was prepared. A topsheet of the prepared diaper was peeled away by curing a hot melt bonding the materials through cold spraying, a topsheet obtained by forming holes through recesses of the peeled uneven topsheet (the same sheet as the topsheet having holes used in Example 5) was arranged and fixed, so that a disposable diaper of Comparative Example 4 was produced.

### Comparative Example 5

A disposable diaper of Comparative Example 5 was produced in a similar way to that of the disposable diaper of Example 1, except that the topsheet having holes used in Example 5 was used as a topsheet and the intermediate sheet used in Comparative Example 2 was used as an intermediate sheet.

### Comparative Example 6

A disposable diaper of Comparative Example 6 was produced in a similar way to that of the disposable diaper of Example 1, except that the topsheet having holes used in Example 5 was used as a topsheet and the intermediate sheet used in Comparative Example 3 was used as an intermediate sheet.

### Loose Stool Evaluation (Dispersion Area / Attachment Amount)

The diapers of Examples 1 to 8 and Comparative Examples 1 to 6 were evaluated as follows. Each diaper was spread out in a planar shape and horizontally placed such that the skin-facing surface side (topsheet side) was oriented upward. Then, 10 g of pseudo-loose stool (viscosity 40 mPa·s) was injected at a time using a metering pump at a flow rate of 6 g/sec to a region distanced 30 mm rearward from the center in the longitudinal direction of the diaper. A liquid impermeable resin film with a size of 10 × 10 cm was placed over the injection position, and a weight was further placed on the resin film, so that the injection position was pressed at 30 gf/cm² for one minute. The load 30 gf/cm² is assumed to be pressure (pressure that is to be withstood) that will be applied to the rear portion of the diaper when a baby that is a few months old and wearing the diaper is sleeping lying face up. Furthermore, the pseudo-loose stool used was a suspension prepared by dissolving or dispersing bentonite in water. The viscosity was adjusted to 40 mPa·s, by adjusting the bentonite concentration. After the pressurization, the weight and the resin film were removed, and the weight of pseudo-loose stool attached to the film was measured. Three measurement samples were prepared for each type of diaper that was to be measured, and were subjected to the above-described measurement. An average of the measured values was taken as the amount of loose stools attached to the diaper. A smaller attachment amount meant better loose stool absorbing performance, which resulted in a higher evaluation result.

Next, after the pressurization, the areas of pseudo-loose stool dispersed on a topsheet and a core-wrap sheet were transferred to OHP sheets, and images thereof were captured by an Image-Pro Plus (manufactured by Nippon Roper K.K.) through scanning. Thus, the area of stools dispersed on each sheet member was obtained. Table 1 shows the results.

**Table 1**

| | | Unit | Ex. 1 | Ex. 2 | Ex. 3 | Ex. 4 | Ex. 5 | Ex. 6 | Ex. 7 | Ex. 8 | Com. Ex. 1 | Com. Ex. 2 | Com. Ex. 3 | Com. Ex. 4 | Com. Ex. 5 | Com. Ex. 6 |
|---|---|---|---|---|---|---|---|---|---|---|---|---|---|---|---|---|
| Topsheet | Whether or not holes are formed | - | Not formed | Not formed | Not formed | Not formed | Formed | Formed | Formed | Formed | Not formed | Not formed | Not formed | Formed | Formed | Formed |
| Intermediate sheet | Basis weight | g/m² | 40 | 40 | 40 | 40 | 40 | 40 | 40 | 40 | - | 40 | 40 | - | 40 | 40 |
| | Shape of non-skin-facing surface | - | Uneven | Uneven | Uneven | Uneven | Uneven | Uneven | Uneven | Uneven | - | Flat | Uneven | - | Flat | Uneven |
| | Adjacent distance between contact portions | mm | 5 | 4 | 5 | 2 | 5 | 4 | 5 | 2 | - | - | 2 | - | - | 2 |
| | Height of internal space | mm | 2.8 | 3.5 | 2.8 | 1.5 | 2.8 | 3.5 | 2.8 | 1.5 | - | - | 1 | - | - | 1 |
| | Non-skin-side contact area percentage | % | 73 | 48 | 40 | 28 | 73 | 48 | 40 | 28 | - | 100 | 88 | - | 100 | 88 |
| Loose stool evaluation (dispersion area) | Topsheet | cm² | 30.0 | 44.0 | 25.5 | 82.2 | 20.1 | 17.0 | 20.8 | 25.3 | 66.8 | 56.6 | 41.7 | 60.8 | 49.3 | 48.2 |
| | Core-wrap sheet | cm² | 31.5 | 48.2 | 27.0 | 87.2 | 28.6 | 86.0 | 86.6 | 35.0 | 67.1 | 55.4 | 40.7 | 61.1 | 48.8 | 46.0 |
| | Dispersion area ratio (core-wrap sheet / topsheet) | - | 1.05 | 1.09 | 1.06 | 1.16 | 1.42 | 2.12 | 1.75 | 1.38 | 1.00 | 0.98 | 0.98 | 1.01 | 1.01 | 1.06 |
| Loose stool evaluation (attachment amount) | | g | 0.61 | 0.64 | 0.56 | 0.60 | 0.46 | 0.37 | 0.48 | 0.61 | 1.28 | 0.95 | 0.81 | 1.10 | 0.89 | 0.88 |

As shown in Table 1, it is seen from a comparison between the amounts of loose stools attached to the diapers of Examples 1 to 8 and the amounts of loose stools attached to the diapers of Comparative Examples 1 to 6 that the amounts of loose stools attached to the disposable diapers of Examples 1 to 8 were smaller than those to the disposable diapers of Comparative Examples 1 to 6. Furthermore, regarding the ratio of the area of stools dispersed on a core-wrap sheet with respect to the area of stools dispersed on a topsheet, the values of the disposable diapers of Examples 1 to 8 were significantly larger than those of the disposable diapers of Comparative Examples 1 to 6, and it is seen that, in the examples, loose stools are likely to be dispersed more on the absorbent member side than on the topsheet side, and return of loose stools to the topsheet can be suppressed. Accordingly, compared with the disposable diapers of Comparative Examples 1 to 6, the disposable diapers of Examples 1 to 8 can effectively absorb loose stools and suppress attachment of loose stools to the wearer's skin, and thus they are expected to reduce occurrence of wearer's skin issues. Moreover, as shown in Table 1, compared with the diapers of Examples 1 to 4 using a topsheet without having holes, the amounts of loose stools attached to the diapers of Example 5 to 8 using a topsheet having holes were significantly smaller, and the values of the ratio of the area of stools dispersed on a core-wrap sheet with respect to the area of stools dispersed on a topsheet were larger, and thus it is seen that, in these examples, loose stools can be more effectively absorbed, and attachment of loose stools to the wearer's skin can be more reliably suppressed.

### Industrial Applicability

According to the absorbent article according to the present invention, it is possible to effectively absorb loose stools, and suppress attachment of loose stools to the wearer's skin.

## Claims

1. An absorbent article comprising a liquid permeable topsheet (2), a liquid retentive absorbent member (4), and a liquid permeable intermediate sheet (5) arranged between the topsheet (2) and the absorbent member (4),
wherein a non-skin-facing surface of the intermediate sheet (5) has a plurality of contact portions (54S) that are in contact with the absorbent member (4) adjacent to the intermediate sheet (5), and a non-contact portion (54N) that is not in contact with the absorbent member (4), wherein a skin-facing surface of the intermediate sheet (5) has skin-side non-contact portions (53N) that are recessed so as to project toward the non-skin-facing surface side, and that is not in contact with the topsheet (2), and
when the intermediate sheet (5) is pressed from the absorbent member side in a thickness direction at a pressure of 30 gf/cm², a proportion of a total area of contact faces, on which the plurality of contact portions located on a pressed face that has been pressed are in contact with the absorbent member, with respect to the pressed face, is 80% or less, wherein the contact area percentage is measured with a method as described in paragraphs [0036] and [0037] of the A1-publication,
wherein topsheet (2) is formed from a layered member of a first sheet (21) and a second sheet (22), wherein the topsheet (2) has a plurality of joined regions (23) formed by partially joining the first sheet (21) and the second sheet (22) the layered member, wherein the joined regions (23) of the topsheet (2) have holes (25) that extend through the joined regions (23), and the holes (25) and the skin-side non-contact portions (53N) overlap each other over an area of 20% or more, and wherein the percentage of holes (25) that overlap, over an area of 20% or more, the skin-side non-contact portions (53N) is 30% or more with respect to all holes (25).

2. The absorbent article according to claim 1, wherein, in a plan view of the absorbent article, the plurality of contact portions are separate from each other in one direction.

3. The absorbent article according to claim 1, wherein, in a plan view of the absorbent article, the plurality of contact portions are separate from each other in two directions.

4. The absorbent article according to any one of claims 1 to 3,
wherein a skin-facing surface of the intermediate sheet has skin-side contact portions that are in contact with the topsheet, and
when the intermediate sheet is pressed from the topsheet side in the thickness direction at a pressure of 30 gf/cm², a proportion of a total area of contact faces, on which the plurality of skin-side contact portions located on a pressed face that has been pressed are in contact with the topsheet, with respect to the pressed face, is larger than the proportion of the total area of the contact faces, on which the contact portions on the non-skin-facing surface side are in contact with the absorbent member, with respect to the pressed face.

5. The absorbent article according to any one of claims 1 to 4,
wherein the first sheet projects in a direction away from the second sheet in regions other than the joined regions, and forms a plurality of projections projecting toward the wearer's skin-side, and
a non-skin-facing surface of the second sheet has a flat face.

6. The absorbent article according to any one of claims 1 to 5, wherein the holes of the topsheet and the skin-side non-contact portion of the intermediate sheet overlap each other in the thickness direction.

7. The absorbent article according to any one of claims 1 to 6,
wherein, on the skin-facing surface of the intermediate sheet, skin-side contact portions that are in contact with the topsheet are separate from each other in both of a longitudinal direction and a lateral direction, and
each of the skin-side contact portions is surrounded by a skin-side non-contact portion that is not in contact with the topsheet.

8. The absorbent article according to any one of claims 1 to 7, wherein, when the intermediate sheet is pressed from the absorbent member side in the thickness direction at a pressure of 30 gf/cm², a non-skin-side contact area percentage, which is a proportion of a total area of contact faces, on which the plurality of contact portions located on a pressed face that has been pressed are in contact with the absorbent member, with respect to the pressed face, is from 20 to 80%.

9. The absorbent article according to claim 8, wherein, when the intermediate sheet is pressed from the topsheet side in the thickness direction at a pressure of 30 gf/cm², a ratio of a skin-side contact area percentage with respect to the non-skin-side contact area percentage is from 1.1 to 10, the skin-side contact area percentage being a proportion of a total area of contact faces on which the plurality of skin-side contact portions located on a pressed face that has been pressed are in contact with the topsheet, with respect to the pressed face.

10. The absorbent article according to claim 9, wherein the skin-side contact area percentage is from 20 to 90%.

11. The absorbent article according to any one of claims 1 to 10, including a front portion that is located on a wearer's ventral side, a rear portion that is located on a dorsal side when worn, and a crotch portion that is positioned between the front portion and the rear portion, and having a longitudinal direction extending from the front portion via the crotch portion to the rear portion and corresponding to a front-rear direction of a wearer, and a lateral direction orthogonal to the longitudinal direction,
the intermediate sheet is rectangular in plan view, has a lengthwise direction thereof matching the longitudinal direction, and covers 30 to 70% of a skin-facing surface of the absorbent member.

## Patentansprüche

1. Absorbierender Artikel, der eine flüssigkeitsdurchlässige Decklage (2), ein flüssigkeitszurückhaltendes absorbierendes Element (4) und eine flüssigkeitsdurchlässige Zwischenlage (5) aufweist, die zwischen der Decklage (2) und dem absorbierenden Element (4) angeordnet ist,
wobei eine nicht-hautzugewandte Oberfläche der Zwischenlage (5) mehrere Kontaktabschnitte (54S), die benachbart zur Zwischenlage (5) in Kontakt mit dem absorbierenden Element (4) sind, und einen Nicht-Kontaktabschnitt (54N) aufweist, der nicht mit dem absorbierenden Element (4) in Kontakt steht, wobei eine hautzugewandte Oberfläche der Zwischenlage (5) hautseitige Nicht-Kontaktabschnitte (53N) aufweist, die so vertieft sind, dass sie zur nicht-hautzugewandten Oberflächenseite vorstehen, und die nicht mit der Decklage (2) in Kontakt stehen, und
wenn die Zwischenlage (5) von der Seite des absorbierenden Elements in einer Dickenrichtung mit einem Druck von 30 gf/cm² gepresst wird, ein Anteil einer Gesamtfläche von Kontaktflächen, auf denen die mehreren Kontaktabschnitte, die sich auf einer gepressten Fläche befinden, die gepresst worden ist, in Kontakt mit dem absorbierenden Element stehen, in Bezug auf die gepresste Fläche 80% oder weniger beträgt, wobei der Kontaktflächenprozentsatz mit einem Verfahren gemessen wird, wie es in den Absätzen [0036] und [0037] der A1-Veröffentlichung beschrieben ist,
wobei die Decklage (2) aus einem geschichteten Element aus einer ersten Lage (21) und einer zweiten Lage (22) ausgebildet ist, wobei die Decklage (2) mehrere verbundene Bereiche (23) aufweist, die durch teilweises Verbinden der ersten Lage (21) und der zweiten Lage (22) des geschichteten Elements ausgebildet sind, wobei die verbundenen Bereiche (23) der Decklage (2) Löcher (25) aufweisen, die sich durch die verbundenen Bereiche (23) erstrecken, und die Löcher (25) und die hautseitige Nicht-Kontaktabschnitte (53N) einander über einen Bereich von 20% oder mehr überlappen, und wobei der Prozentsatz der Löcher (25), die über einen Bereich von 20% oder mehr die hautseitigen Nicht-Kontaktabschnitte (53N) überlappen, 30% oder mehr in Bezug auf alle Löcher (25) beträgt.

2. Absorbierender Artikel nach Anspruch 1, wobei in einer Draufsicht auf den absorbierenden Artikel die mehreren Kontaktabschnitte in einer Richtung voneinander getrennt sind.

3. Absorbierender Artikel nach Anspruch 1, wobei in einer Draufsicht auf den absorbierenden Artikel die mehreren Kontaktabschnitte in zwei Richtungen voneinander getrennt sind.

4. Absorbierender Artikel nach einem der Ansprüche 1 bis 3,
wobei eine hautzugewandte Oberfläche der Zwischenlage hautseitige Kontaktabschnitte aufweist, die in Kontakt mit der Decklage stehen, und
wenn die Zwischenlage von der Seite der Decklage in der Dickenrichtung mit einem Druck von 30 gf/cm² gepresst wird, ein Anteil der Gesamtfläche der Kontaktflächen, auf denen die mehreren hautseitigen Kontaktabschnitte, die sich auf einer gepressten Fläche befinden, die gepresst worden ist, mit der Decklage in Kontakt sind, in Bezug auf die gepresste Fläche größer als der Anteil der Gesamtfläche der Kontaktflächen, auf denen die Kontaktabschnitte auf der Seite der nicht hautzugewandten Oberfläche in Kontakt mit dem absorbierenden Element stehen, in Bezug auf die gepresste Fläche ist.

5. Absorbierender Artikel nach einem der Ansprüche 1 bis 4,
wobei die erste Lage in einer Richtung weg von der zweiten Lage in anderen Bereichen als den verbundenen Bereichen vorsteht und mehrere Vorsprünge bildet, die zur Hautseite des Trägers vorstehen, und
eine nicht hautzugewandte Oberfläche der zweiten Lage eine flache Seite aufweist.

6. Absorbierender Artikel nach einem der Ansprüche 1 bis 5, wobei sich die Löcher der Decklage und der hautseitige Nicht-Kontaktabschnitt der Zwischenlage in der Dickenrichtung überlappen.

7. Absorbierender Artikel nach einem der Ansprüche 1 bis 6,
wobei auf der hautzugewandten Oberfläche der Zwischenlage hautseitige Kontaktabschnitte, die in Kontakt mit der Decklage stehen, sowohl in einer Längsrichtung als auch in einer Querrichtung voneinander getrennt sind, und
jeder der hautseitigen Kontaktabschnitte von einem hautseitigen Nicht-Kontaktabschnitt umgeben ist, der nicht in Kontakt mit der Decklage steht.

8. Absorbierender Artikel nach einem der Ansprüche 1 bis 7, wobei, wenn die Zwischenlage von der Seite des absorbierenden Elements in der Dickenrichtung mit einem Druck von 30 gf/cm² gepresst wird, ein nicht-hautseitige Kontaktflächen-Prozentsatz, der ein Anteil der Gesamtfläche der Kontaktflächen ist, auf der die mehreren Kontaktabschnitte, die sich auf einer gepressten Fläche befinden, die gepresst worden ist, in Kontakt mit dem absorbierenden Element stehen, in Bezug auf die gepresste Fläche 20 bis 80% beträgt.

9. Absorbierender Artikel nach Anspruch 8, wobei, wenn die Zwischenlage von der Oberlagenseite in der Dickenrichtung mit einem Druck von 30 gf/cm² gepresst wird, ein Verhältnis eines hautseitigen Kontaktflächen-Prozentsatzes in Bezug auf den nichthautseitigen Kontaktflächen-Prozentsatz von 1,1 bis 10 beträgt, wobei der hautseitige Kontaktflächen-Prozentsatz ein Anteil einer Gesamtfläche von Kontaktflächen, auf denen die mehreren hautseitigen Kontaktabschnitte, die sich auf einer gepressten Fläche befinden, die gepresst worden ist, in Kontakt mit der Decklage sind, in Bezug auf die gepresste Fläche ist.

10. Absorbierender Artikel nach Anspruch 9, wobei der der hautseitige Kontaktflächen-Prozentsatz zwischen 20 und 90% liegt.

11. Absorbierender Artikel nach einem der Ansprüche 1 bis 10, der einen vorderen Abschnitt, der sich auf der Bauchseite eines Trägers befindet, einen hinteren Abschnitt, der sich beim Tragen auf der Rückenseite befindet, und einen Schrittabschnitt aufweist, der zwischen dem vorderen Abschnitt und dem hinteren Abschnitt angeordnet ist, und eine Längsrichtung, die sich vom vorderen Abschnitt über den Schrittabschnitt zum hinteren Abschnitt erstreckt und einer Vorne-Hinten-Richtung eines Trägers entspricht, und eine Querrichtung orthogonal zur Längsrichtung aufweist,
die Zwischenlage in der Draufsicht rechteckig ist, eine Längenrichtung davon aufweist, die mit der Längsrichtung übereinstimmt, und 30 bis 70% einer hautzugewandten Oberfläche des absorbierenden Elements bedeckt.

## Revendications

1. Article absorbant, comprenant une feuille supérieure (2) perméable aux liquides, un élément absorbant (4) retenant les liquides, et une feuille intermédiaire (5) perméable aux liquides disposée entre la feuille supérieure (2) et l'élément absorbant (4),
où une surface non exposée à la peau de la feuille intermédiaire (5) comporte une pluralité de parties de contact (54S) en contact avec l'élément absorbant (4) adjacent à la feuille intermédiaire (5), et une partie exempte de contact (54N) n'étant pas en contact avec l'élément absorbant (4), où une surface exposée à la peau de la feuille intermédiaire (5) comporte des parties exemptes de contact côté peau (53N), ménagées de manière à être en saillie vers le côté de la surface non exposée à la peau, et n'est pas en contact avec la feuille supérieure (2), et
où, quand la feuille intermédiaire (5) est comprimée dans le sens de l'épaisseur depuis le côté d'élément absorbant avec une pression de 30 gf/cm², la proportion de la surface totale des faces de contact, sur laquelle la pluralité de parties de contact présentées sur une face ayant été comprimée sont en contact avec l'élément absorbant, par rapport à la face comprimée, est égale ou inférieure à 80 %, le pourcentage de surface de contact étant mesuré suivant un procédé décrit aux paragraphes [0036] et [0037] de la publication A1, où la feuille supérieure (2) est formée par un élément stratifié d'une première feuille (21) et d'une deuxième feuille (22), où la feuille supérieure (2) comporte une pluralité de zones liées (23) formées par liaison partielle de la première feuille (21) et de la deuxième feuille (22) de l'élément stratifié, où les zones liées (23) de la feuille supérieure (2) présentent des trous (25) traversant les zones liées (23), et les trous (25) et les parties exemptes de contact côté peau (53N) se chevauchent sur une surface de 20 % ou plus, et où le pourcentage de trous (25) chevauchant sur une surface de 20 % ou plus les parties exemptes de contact côté peau (53N) est égal ou supérieur à 30 % par rapport à l'ensemble des trous (25).

2. Article absorbant selon la revendication 1, où, en vue en plan dudit article absorbant, la pluralité de parties de contact sont séparées l'une de l'autre dans une direction.

3. Article absorbant selon la revendication 1, où, en vue en plan dudit article absorbant, la pluralité de parties de contact sont séparées l'une de l'autre dans deux directions.

4. Article absorbant selon l'une des revendications 1 à 3,
où la surface exposée à la peau de la feuille intermédiaire comporte des parties de contact côté peau en contact avec la feuille supérieure, et
où, quand la feuille intermédiaire est comprimée dans le sens de l'épaisseur depuis le côté de feuille supérieure avec une pression de 30 gf/cm², la proportion de la surface totale des faces de contact, sur laquelle la pluralité de parties de contact côté peau présentées sur une face ayant été comprimée sont en contact avec la feuille supérieure, par rapport à la face comprimée, est supérieure à la proportion de la surface totale des faces de contact, sur laquelle les parties de contact sur le côté de la surface non exposée à la peau sont en contact avec l'élément absorbant, par rapport à la face comprimée.

5. Article absorbant selon l'une des revendications 1 à 4,
où la première feuille fait saillie dans une direction d'éloignement de la deuxième feuille dans des zones autres que les zones liées, et forme une pluralité de saillies s'étendant vers le côté peau du porteur, et
une surface non exposée à la peau de la deuxième feuille a une face plane.

6. Article absorbant selon l'une des revendications 1 à 5, où les trous de la feuille supérieure et la partie exempte de contact côté peau de la feuille intermédiaire se chevauchent dans le sens de l'épaisseur.

7. Article absorbant selon l'une des revendications 1 à 6,
où, sur la surface exposée à la peau de la feuille intermédiaire, des parties de contact côté peau en contact avec la feuille supérieure sont séparées l'une de l'autre dans une direction longitudinale et une direction latérale, et
chacune des parties de contact côté peau est entourée par une partie exempte de contact côté peau n'étant pas en contact avec la feuille supérieure.

8. Article absorbant selon l'une des revendications 1 à 7, où, quand la feuille intermédiaire est comprimée dans le sens de l'épaisseur depuis le côté d'élément absorbant avec une pression de 30 gf/cm², un pourcentage de surface de contact côté autre que peau, qui est la proportion de la surface totale des faces de contact, sur laquelle la pluralité de parties de contact présentées sur une face ayant été comprimée sont en contact avec l'élément absorbant, par rapport à la face comprimée, est compris entre 20 et 80 %.

9. Article absorbant selon la revendication 8, où, quand la feuille intermédiaire est comprimée dans le sens de l'épaisseur depuis le côté de feuille supérieure avec une pression de 30 gf/cm², le rapport entre un pourcentage de surface de contact côté peau et le pourcentage de surface de contact côté autre que peau est compris entre 1,1 et 10, le pourcentage de surface de contact côté peau étant la proportion de la surface totale de faces de contact sur laquelle la pluralité de parties de contact côté peau présentées sur une face ayant été comprimée sont en contact avec la feuille supérieure, par rapport à la face comprimée.

10. Article absorbant selon la revendication 9, où le pourcentage de surface de contact côté peau est compris entre 20 et 90 %.

11. Article absorbant selon l'une des revendications 1 à 10, présentant une partie avant disposée sur un côté ventral du porteur, une partie arrière disposée sur un côté dorsal pendant lorsque l'article est porté, et une partie d'entrejambe entre la partie avant et la partie arrière, et présentant une direction longitudinale s'étendant de la partie avant à la partie arrière par la partie d'entrejambe et correspondant à la direction de l'avant vers l'arrière du porteur, et une direction latérale orthogonale à la direction longitudinale, la feuille intermédiaire étant rectangulaire en vue en plan, le sens de la longueur de celle-ci correspondant à la direction longitudinale, et couvrant de 30 à 70 % de la surface exposée à la peau de l'élément absorbant.
